# EUROPEAN PATENT SPECIFICATION

(11) **EP 4 370 072 B1**
(45) Date of publication and mention of the grant of the patent: **16.04.2025**
(21) Application number: 22750719.1
(22) Date of filing: 14.07.2022
(51) Int. Cl.: A61F 2/58, A61F 2/70, A61F 2/72, A61F 2/76, A61F 2/50

(54) **ARTIFICIAL HAND**
KÜNSTLICHE HAND
MAIN ARTIFICIELLE

(30) Priority: 14.07.2021 GB 202110130
(43) Date of publication of application: 22.05.2024
(73) Proprietor: Kingswell, Alan James Attard, Ta Giorni, St. Julians (MT)
(72) Inventor: Kingswell, Alan James Attard, Ta Giorni, St. Julians (MT)
(74) Representative: J A Kemp LLP
(86) International application number: PCT/EP2022/069780
(87) International publication number: WO 2023/285611

(56) References cited:
- WO-A1-2019/156643
- WO-A2-2011/036626
- WO-A2-2014/111843
- CN-A- 111 419 489
- US-A1- 2014 163 731

## Description

### BACKGROUND OF THE INVENTION

This invention relates to artificial hands, suitable for use as prosthetic or robotic hands, also sometimes termed bionic hands.

Prosthetic hands are provided to persons who lack natural hands owing to amputation because of trauma, illnesses, or congenital defects, to afford desirable capability in the performance of manual actions such as grasping and moving objects, if not the complete dexterity of natural hand movements. Typically or preferably, prosthetic hands are mechanically integral (not requiring an associated artificial arm), operated with a power supply rather than body powered, and configured to resemble a natural hand. As is well known in the art, present day prosthetic hands commonly are employed with myoelectric control.

At present, advanced electronic prosthetic hands, though achieving high dexterity, are very high in cost and therefore not accessible to many potential users.

WO 2011/036626 A2 discloses an orientation controller, mechanical arm, gripper and components thereof.

### SUMMARY OF THE INVENTION

An object of the present invention is to provide new and improved artificial hands, including hands suitable for prosthetic hands and hands suitable for robotic use. Additional objects are to provide a natural looking, motorized and electronically controllable prosthetic hand with a high level of dexterity, integral mechanism, good strength and low cost, capable of being articulated into many positions for grasping different objects and performing different functions and gestures, including most common motions, such as grip patterns that require all digits to wrap around an object as well as pointing and pinching, which require other digits to be pre-closed and thumb and pointer finger to align.

The invention is as defined in claim 1. Further aspects and preferred embodiments are defined in the dependent claims. Any aspects, embodiments and examples of the present disclosure which do not fall under the scope of the appended claims do not form part of the invention and are merely provided for illustrative purposes.

Further features and advantages of the invention will be apparent from the detailed description set forth below, together with the accompanying drawings.

### BRIEF DESCRIPTION OF THE DRAWINGS

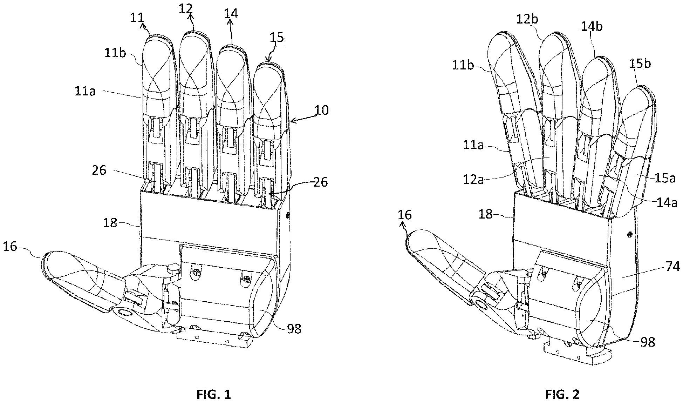

FIG. 1 is a perspective palm view of an artificial hand, in particular a prosthetic hand, embodying the present invention in a particular form, showing the finger digits in adducted relation and the thumb digit in a first position;
FIG. 2 is a perspective palm view of the same prosthetic hand, showing the finger digits in abducted relation and the thumb digit in a second position;
FIG. 3 is a view of one side of the same prosthetic hand;
FIG. 4 is a back view of the same prosthetic hand;
FIG. 5 is a view of the opposite side of the same prosthetic hand;
FIGS. 6 and 7 are back views of the same prosthetic hand with the finger digits respectively in adducted and abducted positions:

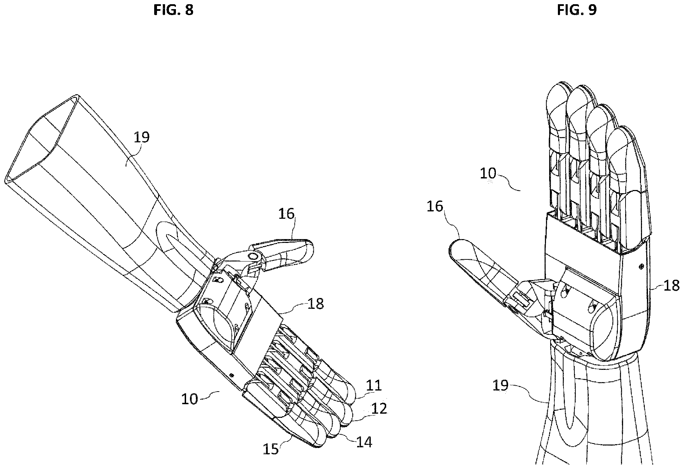

FIGS. 8 and 9 are perspective palm views of the same prosthetic hand with an attached forearm socket also usable as a display stand;

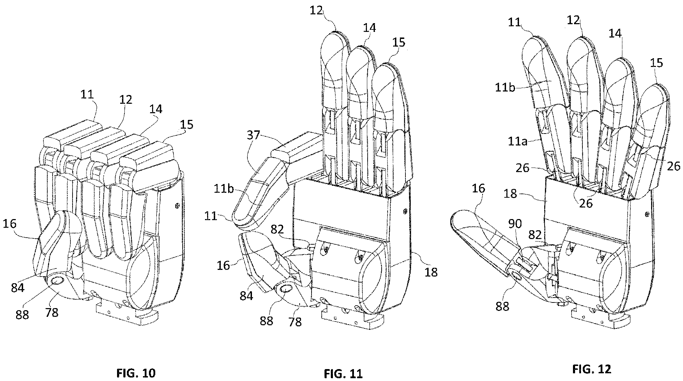

FIGS. 10, 11 and 12 are perspective palm views of the same prosthetic hand, respectively showing the finger and thumb digits in clenched position, the index finger and thumb digits positioned to grasp or pinch an object between them, and the thumb and finger digits open (abducted);
FIGS. 13, 14 and 15 are similar views of the same prosthetic hand showing further positions of the index finger digit;

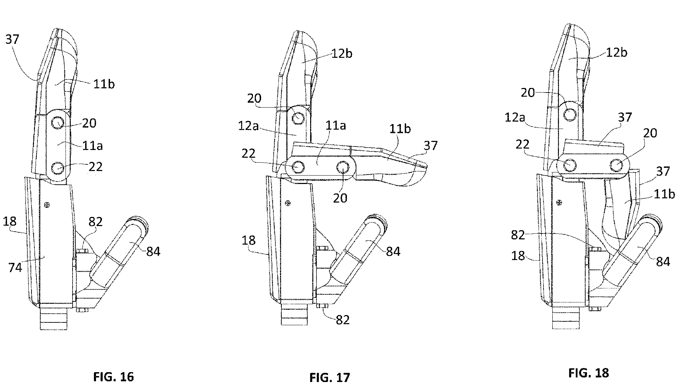

FIGS. 16, 17 and 18 are side views of the same prosthetic hand with finger digits in the same positions as FIGS. 13, 14 and 15 respectively;
FIGS. 19 and 20 are perspective palm views of the same prosthetic hand with the palm body casing removed, respectively showing the middle finger digit and its associated linear actuator in two different positions;
FIG. 21 is a perspective view of the linear actuator for one of the finger digits of the same prosthetic hand;
FIGS. 22A and 22B are, respectively, plan views of the actuator of FIG. 21 with its driver member fully retracted (finger digit fully inwardly curled) and fully extended (finger digit fully straight);
FIG. 23 is a sectional view of the same prosthetic hand, taken along the length of the index finger digit through that digit and its associated linear actuator, with that finger digit fully extended;
FIG. 24 is a sectional view similar to FIG. 23 with the index finger digit fully curled;
FIGS. 25 and 26 are perspective views of the back of the same prosthetic hand with the palm body casing removed, illustrating the mechanism for moving the finger digits between adducted (FIG. 25) and abducted (FIG. 26) relation to each other;
FIGS. 27 and 28 are back views of the same prosthetic hand respectively corresponding to FIGS. 25 and 26;
FIGS. 29 and 30 are fragmentary palm views of the same prosthetic hand with the palm boy casing removed, showing the middle finger digit in adducted and abducted positions, respectively;
FIGS. 31 and 32 are enlarged views of the same middle finger digit respectively corresponding to FIGS. 29 and 30;
FIGS. 33 and 34 are exploded perspective palm views of the same prosthetic hand illustrating successive stages in assembling the hand;
FIG. 35 is a perspective palm view of the same hand when fully assembled;
FIGS. 36 and 37 are perspective views, from different directions, of the thumb digit assembly of the same prosthetic hand;
FIG. 38 is a further perspective view of the same thumb digit assembly;
FIG. 39 is a partially sectional view of the same thumb digit assembly;
FIGS. 40, 41 and 42 are perspective palm views of the same prosthetic hand illustrating different positions of the thumb digit;
FIGS. 43, 44 and 45 are end views of the same prosthetic hand, respectively showing the thumb digit positions of FIGS. 40, 41 and 42, and
FIGS. 46, 47 and 48 are side views of the same prosthetic hand, also respectively showing the thumb digit positions of FIGS. 40, 41 and 42,

### DETAILED DESCRIPTION

For purposes of illustration, the invention will be described as embodied in an artificial hand, in particular suitable for use as a prosthetic hand 10 (here shown as a left hand) having four finger digits 11, 12, 14 and 15 respectively corresponding to the index, middle, ring and little fingers of a natural human hand, a thumb digit 16, and a palm body 18 on which the finger and thumb digits are pivotally mounted in an arrangement simulating the positions of the four fingers, thumb and palm of the natural hand. These features are constituted of rigid elements conveniently or preferably fabricated of a suitable plastic by three-dimensional (3D) printing, as hereinafter further discussed (alternatively, the elements may be made using any other 3D printing or traditional manufacturing technology, such as CNC machines, casting, molding, etc.). The finger digits, thumb digit and palm body may have relative lengths generally conforming to their human hand counterparts. If desired, the prosthetic hand may be provided with a socket forearm 19 (FIGS, 8 and 9) also usable as a display stand.

Each of the finger digits comprises two phalange sections, viz. a proximal phalange section (corresponding to the proximal phalange of a human finger) and a distal phalange section (corresponding to the intermediate and distal phalanges of a human finger as if fused together with a slight bend between them to facilitate wrapping around objects). In the index finger digit, the proximal phalange section is designated 11a and the distal phalange section is designated 11b; in the middle finger digit, the proximal and distal phalange sections are 12a and 12b respectively; in the ring finger digit, the two phalange sections are respectively 14a and 14b; and in the little finger digit, the two phalange sections are respectively 15a and 15b. The combination of distal and middle phalanges in a single rigid distal phalange section serves to simplify the construction of the prosthetic hand and enhance durability, as it is found that a pivotal connection between distal and middle phalanges is not strictly necessary for satisfactory functioning of the finger digits. If desired, however, these fused distal phalange sections may be replaced with separate distal and middle phalange sections interconnected by a pivot joint.

In each finger digit, the distal end of the proximal phalange section and the proximal end of the distal phalange section are interconnected by a pivot pin 20, which enables relative rotation of the two phalange sections about a pivot axis that is perpendicular to the long dimension of the finger digit when the latter is fully extended. Similarly, the proximal end of the proximal phalange section of each finger digit is connected to the distal end of the palm body 18 by a pivot pin 22 which enables rotation of the proximal phalange section relative to the palm body about a pivot axis parallel to the axis defined by pivot pin 20. The proximal and distal phalange sections of each finger digit are, therefore, pivotally movable relative to each other and to the palm body in simulation of movement of a human finger between extended and inwardly curled positions relative to a human palm.

More particularly, in the described embodiment the proximal phalange sections 11a, 12a, 14a and 15a of the four finger digits are respectively mounted to the palm body through knuckle elements 11c, 12c, 14c and 15c. That is, the pivot pins 22 respectively connect the proximal phalange sections to the knuckle elements. Each knuckle element is connected by a pivot pin 24 to the palm body for rotation relative thereto in a plane parallel to the axis of rotation of the associated proximal phalange section about its pin 22 relative to the knuckle element. If the axes of rotation about pins 20 and 22 are horizontal, the axes of rotation about pins 24 are vertical.

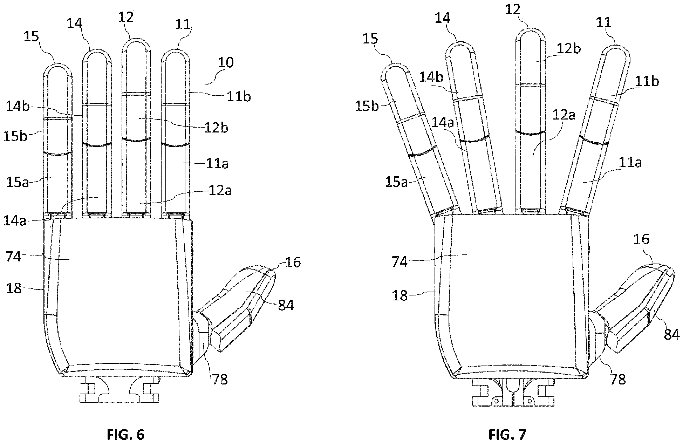

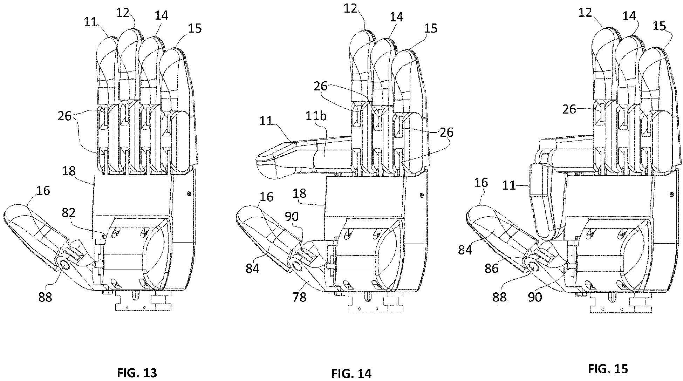

Features such as mating surfaces of the phalange sections may be adjusted to generate different frictional forces upon application of pivotal forces such that the proximal phalange section of each finger digit pivots (relative to the palm body) before the distal phalange section, as is desired for object grasping and enhanced control (FIGS. 13-15). In addition, each digit has slightly different lengths for each section (FIG. 6), for improved gripping effect and natural appearance. Optionally, soft rubber grip pads (not shown) may be added on the tips and internal faces of the digits, to help grip smooth objects.

As in some known prosthetic hands, the present invention provides tendons for the finger digits to retract or curl the digits from extended position. While other prosthetic hand designs have employed direct mechanical linkages to flex the digit joints, such arrangements are costly, difficult to incorporate in the structure without reduced flexibility, lessened gripping and holding strength, and vulnerability to damage, e.g. breakage of fingers..

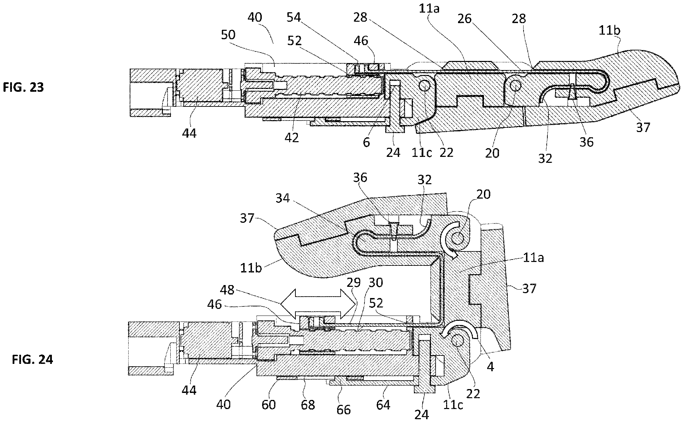

In accordance with the invention, in the embodiment shown, as a tendon for each of the finger digits, there is provided an elongated flexible flat strap member 26 extending lengthwise through slots or channels 28 formed in the successive phalange sections (e.g. sections 11a and 11b of index finger digit 11). As thus disposed, each strap member has major flat surfaces 29, 30 substantially parallel to the axes of rotation about the pivot pins 20 and 22 of its associated finger digit (see FIG. 23) but offset inwardly therefrom with respect to a direction of finger digit curling toward the palm face 18a of the palm body 18, the strap member further having a distal end 32 anchored to the distal phalange section (e.g. section 11b). Specifically, strap member distal end 32 may be wrapped around an internal post 34 in the distal phalange section and anchored by a self-tapping retaining screw 36 which passes through the plastic of the latter phalange section and the strap.

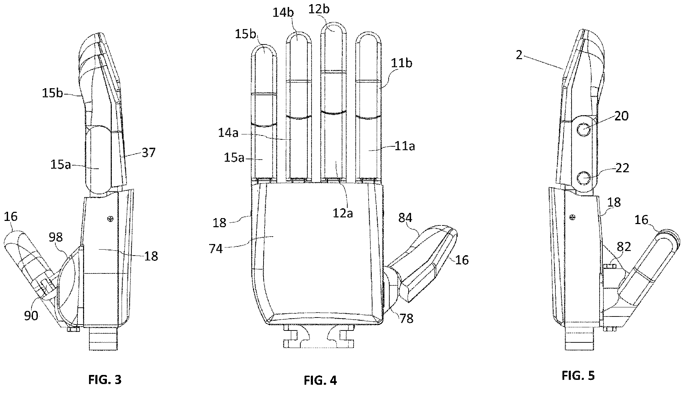

Each finger digit may have a plate 37 (FIG. 3) that may (for example) fit in a friction fit on the back of each phalange section, the end portion of this plate serving to cover the retaining screw 36. The provision of plate 37 also facilitates 3D printing of the finger digits and may add a decorative effect if printed in a different color.

Owing to the inwardly offset position of each tendon or strap member with respect to the rotational axes defined by pins 20 and 22 in its associated finger digit, pulling of the strap member in a proximal direction from its proximal end causes the finger digit to curl or close on itself (FIG. 23), since the length of the portion of the strap member distally of the pivot pin 22 is thereby decreased, forcing the phalange sections to rotate around the pivot pin axes to bring them closer together.

Illustrative but non-limiting examples of articles suitable for use as the strap members 26 of the invention are the currently commercially available products known as zip ties or cable ties made of nylon. These ties have a rectangular strap cross-section of such proportions that they are flexible primarily in one direction while being much more rigid laterally as well as in compression and tension; hence they can curl on themselves to actuate (bend inwardly) a finger digit 11, 12, 14 or 15 when pulled from their proximal (actuating driven) ends 38 (see FIG. 24) and can also be pushed from their same ends 38 to straighten a bent finger digit. For instance, in a specific example for a typical adult-sized prosthetic hand, the strap members may be nylon zip ties or cable ties each having a length of about 150 mm (trimmed down after being secured), a transverse width of about 2.5 mm and thickness of e.g. about 0.9 mm.

Use of a strap member as a tendon thus enables the tendon to effect extension of a curled finger digit as well as curling of an extended finger digit, merely by having the proximal end of the strap member pushed longitudinally by the same actuator that curls the digits by pulling the same end of the strap member. This obviates the provision of spring-biasing means or other resilient arrangements for re-straightening a curled finger digit, as has heretofore been necessary with tendons made of string or the like. Not only is the structure of the prosthetic hand thereby simplified, but in addition the force required to retract (curl) an extended finger is reduced, because there is no extension-biasing force to be overcome, and the actuator need not be kept energized to maintain the finger digit in curled position.

The use of strap members as tendons for the finger digits also affords an advantageously wide surface area to spread the pressure of the forces being applied to the finger digit material along which the tendon slides when under tension, thereby reducing wear, in contrast to the concentrated and abrasive force of a tendon made of string. In the case of nylon zip ties, the nylon is a self-lubricating material which slides easily within the slots or channels of the phalange sections, as well as being one of the strongest polymers. Moreover, such tendons render the finger digits beneficially compliant in that they enable the digits to flex slightly when subjected to external forces while the actuator is fixed.

Each of the strap members 26 extends proximally past and beyond the knuckle element (e.g. element 11c) of its associated finger digit and into the palm body 18 for attachment to a linear actuator for that finger digit, i.e., an actuator that moves the strap member linearly along a path generally parallel to the associated finger digit. Stated with reference to the index finger digit 11, in accordance with currently preferred embodiments of the invention a linear actuator 40 therefor is disposed within the palm body and connected to the proximal end 38 of the strap member 26 for moving the strap member longitudinally through the phalange sections 11a and 11b of the finger digit 11 in each of two opposed directions thereby to move the phalange sections pivotally relative to each other and to the palm body between positions respectively simulating fully extended and inwardly curled positions of the finger digit.

Each linear actuator 40 in the illustrated embodiment of the invention is shown as comprising a lead screw 42, a DC motor 44 for rotating the lead screw in each of two opposed directions (e.g. a DC metal geared Common N20 Form factor motor), and a driver member 46 threaded on the lead screw for longitudinal movement therealong in each of two opposed directions (indicated by arrow 48) dependent on the direction of rotation of the lead screw, the driver member being anchored to the proximal end 38 of the strap member 20 for moving the strap member longitudinally through the phalange sections.

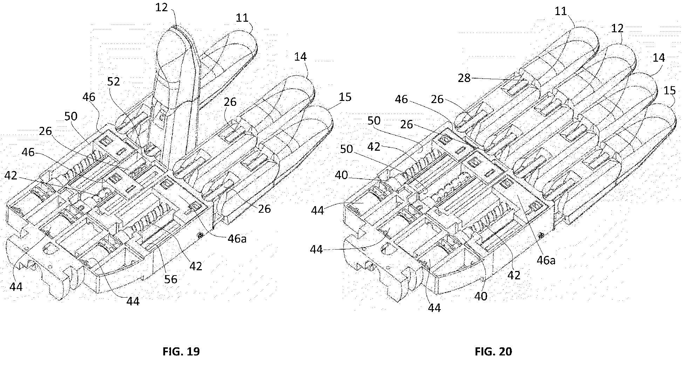

The lead screw 42 of each linear actuator is mounted in a generally rectangular actuator housing 50 entirely within, and constituting part of, the palm body, for rotation about an axis generally parallel to the direction in which the finger digits extend (FIGS. 19 and 20). The driver member 46 threaded thereon is rectangular and constrained against rotation by the side walls of housing 50 so that rotation of the lead screw moves the driver member digitally or proximally depending on the direction of rotation. Through a slit 52 in the front of the actuator housing, the associated strap member 26 extends from the finger digit into the housing, and the proximal end 38 of the strap member overlies and is securely affixed to the driver member, for example utilizing the small rectangular plastic feature 54 conventionally formed at one end of zip ties; thus, the proximal end of the strap member is stacked above the lead screw, providing a compact assembly. Movement of the driver member, when the lead screw turns, pushes or pulls the strap member through the slit 52 to open or close the associated finger digit(s). The backlash of the lead screw holds the digit in any position to which it is moved without need to maintain power to the motor.

It is currently preferred to fabricate the lead screws 42 of plastic by 3D printing in order to facilitate customization of strength/travel distance/speed to actuate, for example in production of child-size devices which have reduced space for linear travel of the driver member but may also be less strong, or to accommodate the choice of individual users with respect to actuation speed vs. strength.

For various grip patterns as well as for determining whether a finger digit has reached its fully extended or fully curled position, each of the linear actuators 40 for the finger digits has a linear potentiometer 56 extending alongside it, to measure the position of the driver member along the lead screw (alternatively, each potentiometer may be disposed directly beneath its associated linear actuator). This feedback provides the absolute position of a wiper (carried by the driver member) along the length of a resistive path to the microcontroller for the hand. The microcontroller can use the supplied data to determine the digit position and if necessary instruct the actuator motor to rotate the lead screw so as to move the driver member to the currently required position for each digit independently. Other means for position sensing (e.g. soft potentiometers, to reduce the width of the device by, say, 10 mm, to accommodate the electronics on board), and other sensors such as force sensitive resistors in the distal phalange tips, may also be employed with the hand.

Although the described embodiment of the prosthetic hand has four finger digits it includes only three finger digit linear actuators 40, arranged side by side in the palm body. There are separate linear actuators for each of the finger digits 11 and 12 respectively corresponding to the index and middle fingers, but the digits 14 and 15 respectively corresponding to the ring and little fingers (which are rarely used independently in a natural hand) share a common linear actuator 40a; the driver member 46a of the actuator 40a is made wide enough to accommodate the proximal ends of the strap members of both digits 14 and 15 mounted thereon. This saves space, reduces the total number of required components, and reduces the electronic control requirements while providing a full complement of finger digits for realistic appearance and grip. Alternatively, if desired, all four finger digits may provided with individual linear actuators so that the ring and little fingers are separately movable between open and closed positions.

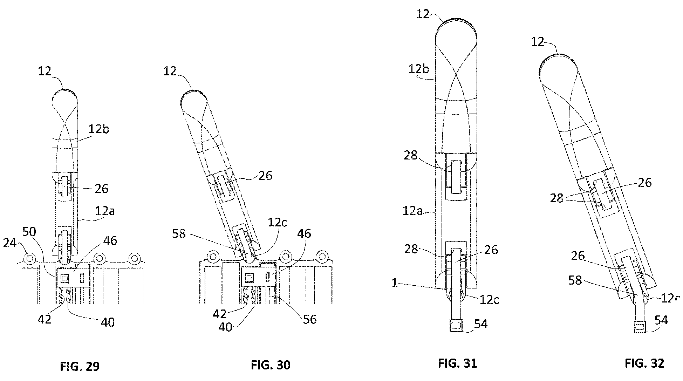

An additional important feature of the present invention in the embodiment shown is the provision of an arrangement for spreading (splaying) the fingers laterally from closed (adducted) position to open (abducted) position, substantially in a common plane. This is enabled by the pivotal mounting of the knuckle elements 11c, 12c, 14c and 15c on the palm body 18 by the pins 24 for limited individual abduction-adduction rotation (up to a total angle of about 60°), relative to the palm body, about axes perpendicular to the axes of rotation of the proximal phalange sections about the pins 22 relative to the knuckle elements. The pins 24 are located on the back of the prosthetic hand while the strap members 26 overlie the palm-facing surfaces of the knuckle elements so that the strap members pass from the proximal phalange sections of the finger digits above the knuckle elements (as seen in FIGS. 23 and 24) to their associated linear actuator driver members 46 without interference from pins 24. The strap members 36 pass over the centerlines of (axes of knuckle rotation about) pins 24 and are guided into the linear actuators through slits 52; hence abduction-adduction rotation of the finger digits does not change the longitudinal distance the strap members need to move in order to pull or push their associated finger digits and does not change the point at which the strap members enter the palm body to be actuated; also, although abductive pivoting of the finger digits requires slight lateral bending of the strap members forwardly of the linear actuators as indicated at 58 (see FIGS. 29-32), the above-described strap members have sufficient lateral flexibility to accommodate the limited extent of lateral bending involved and still be able to be pushed or pulled longitudinally through their associated slits 52 and phalange section channels 28.

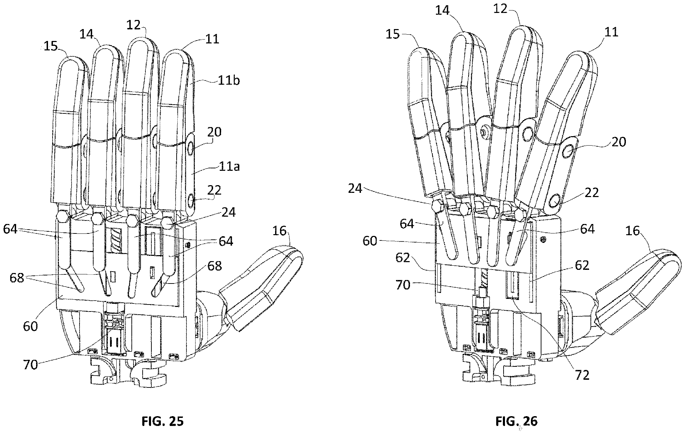

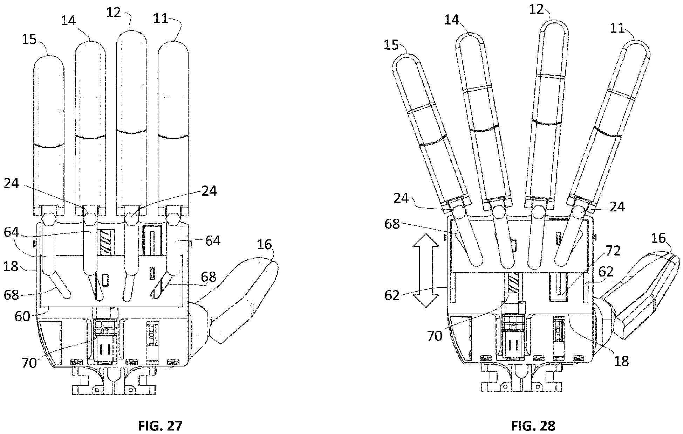

The finger digits are driven together between their adducted (FIGS. 25 and 27) and abducted (FIGS. 26 and 28) positions by a plate 60 mounted in guide grooves 62 on the back of the palm body 18 for sliding motion between a proximal position (FIG. 25) and a distal position (FIG. 26) on the palm body. Each of the knuckle elements 11c, 12c, 14c and 15c has a thin flat arm 64 extending proximally therefrom over the plate 60. At the proximal end of each arm 64 is a thin flat peg 66 projecting toward the plate 60 and inserted in one of four diverging slots 68 formed in the plate such that the pegs of different finger digits are respectively received in different slots. A linear actuator 70, similar to the linear actuators 40 and also mounted in the palm body, is connected to the plate 60 so as to move it between the aforesaid proximal and distal positions; as in the case of the finger digits and actuators 40, the location of the plate between the limits of its proximal-distal travel (and thus the degree of abduction of the finger digits) is controlled by controlling the position of the linear actuator.70, the latter position being measured e.g. with a linear potentiometer 72 or other linear sensing means. It may be noted that the use of a single linear actuator 40 to curl and extend both the ring finger and the little finger digits facilitates the provision of space in the palm body for the linear actuator 70 that drives the adduction and abduction of the finger digits, although it is also possible to fit four separate linear actuators for fingers as well as the actuator 70 into the palm.

In the illustrated embodiment, the four slots 68 in plate 60 diverge in a distal direction. Consequently, when the plate 60 is at its proximal limit of travel (FIGS. 25 and 27), the four pegs 66 (respectively guided by the four slots 68) are located at the distal, maximally separated ends of the slots, and the four knuckle elements 11c, 12c, 14c and 15c are pivoted about their respective pins 24 into positions where the four finger digits are parallel (adducted). Upon movement of plate 60 by linear actuator 70 to the distal limit of plate travel (FIGS. 26 and 28), the pegs 66 are located at the proximal, minimally separated slot ends, pivoting the four finger digits into their maximally separated (fully abducted) positions.

It is not necessary that each digit can be adducted and abducted. For example, the one or both of the central digits 12, 13 may remain stationary. Accordingly, the above described mechanisms may only be present for those digits that are configured to be adducted and abducted.

In the described embodiment of the invention, the palm body has a rigid casing 74 for receiving and enclosing the finger-digit linear actuators 40, the adductor-abductor linear actuator 70, the adductor-abductor plate 60 and the knuckle arms 64. The casing, which may have decorative features (not shown) is held in place by two screws 75. Alternative arrangements may, for example, include additional guiding plates to reduce play of the actuators, and a removable palm face for ease of wire management and repair.

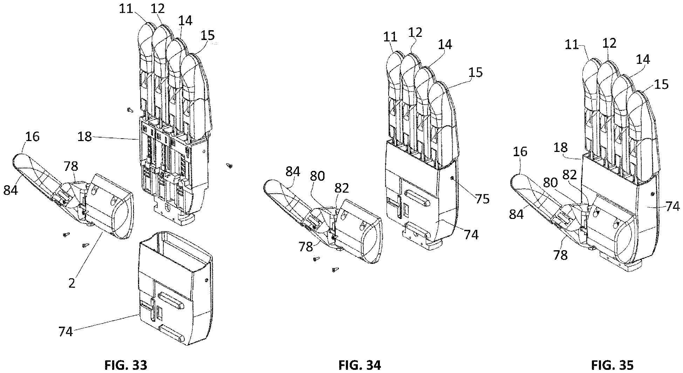

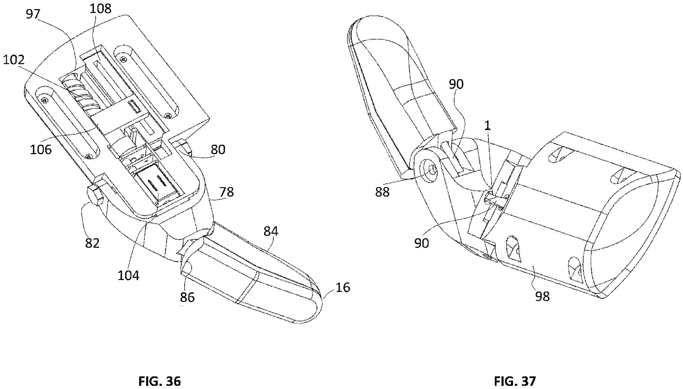

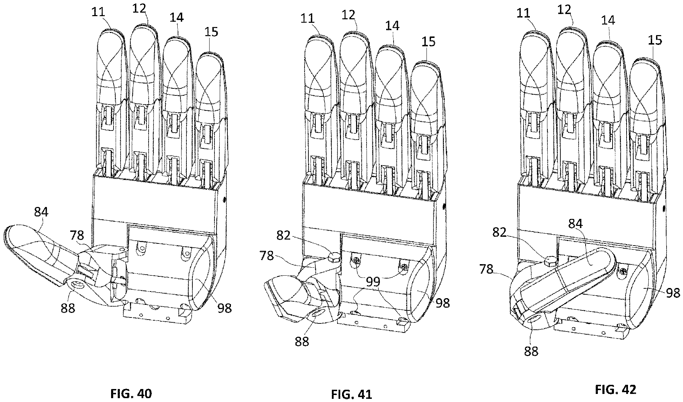

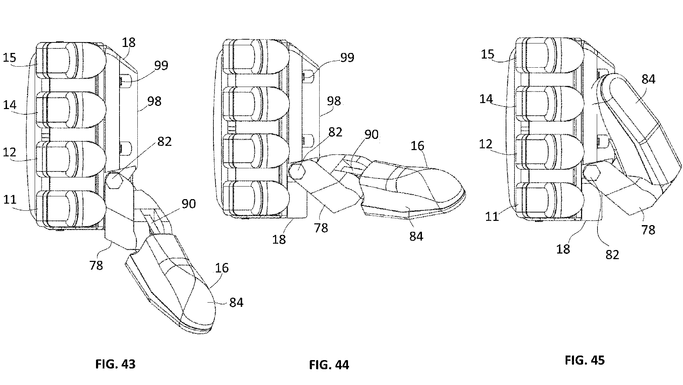

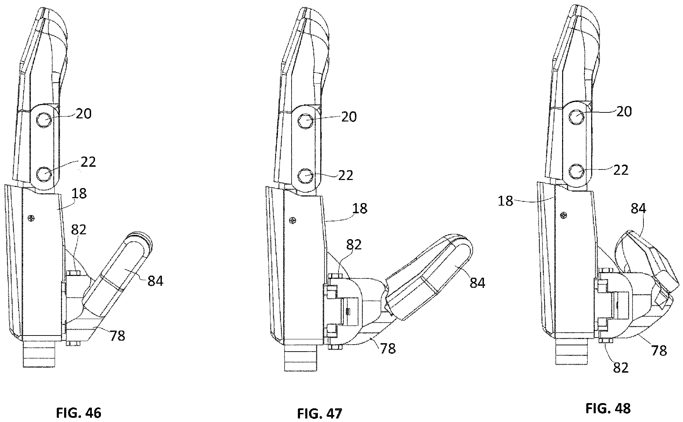

The artificial hand of the invention, in this embodiment, also includes a thumb digit 16 (FIGS. 33-48) mounted on the palm face of the palm body casing and extending laterally from the palm body. The thumb digit has a proximal thumb phalange section 78 with a proximal end 80 pivotally connected by a joint 82 to the palm body for rotation relative thereto about a first thumb axis, and a distal thumb phalange 84 section with a proximal end 86 pivotally connected by a pin 88 to the distal end of the proximal thumb phalange section 78 for rotation relative thereto about a second thumb axis non-parallel to the first thumb axis, such that the thumb phalange sections are pivotally movable relative to each other and to the palm body in simulation of movement of a human thumb relative to a human palm;.

As a tendon for the thumb digit, there is provided an elongated flexible flat thumb strap member 90 extending lengthwise through the successive thumb phalange sections 78, 84 with major flat surfaces 92 and 94 offset inwardly from the aforesaid first and second thumb axes with respect to a direction of thumb bending, the strap member 90 having a distal end anchored to the distal thumb phalange section 84. The successive thumb phalange sections cooperatively define a non-rectilinear path for guiding the thumb strap member longitudinally therethrough. This strap member 90 may be of the same type as the above-described strap members 26 of the finger digits, and thus may (for example) be a nylon zip tie or cable tie, having the same properties already discussed.

A thumb linear actuator 97 is mounted within a housing 98 secured by four screws 99 on the palm body (whereby the thumb digit is mounted on the palm body), and connected to a proximal end 100 of the thumb strap member 90 for moving the thumb strap member longitudinally through the thumb phalange sections in each of two opposed directions thereby to move the thumb phalange sections pivotally relative to each other and to the palm body between positions respectively simulating said extended and inwardly curled positions of a human thumb relative to a human palm. The thumb linear actuator, in the form shown, comprises a lead screw 102 extending transversely across the palm body (perpendicular to the rotational axis of pivot joint or pin 82, a motor 104 for rotating the lead screw in each of two opposed directions, and a driver member 106 threaded on the lead screw for longitudinal movement therealong in each of two opposed directions dependent on the direction of rotation of the lead screw. The driver member 106 is anchored to the proximal end 100 of the thumb strap member 90 for moving the thumb strap member longitudinally through the thumb phalange sections as aforesaid. The structure and operation of the linear actuator 90 may be the same as that of the aforementioned linear actuators 40 and 70, and for control purposes may be provided in like manner with a linear potentiometer 108.

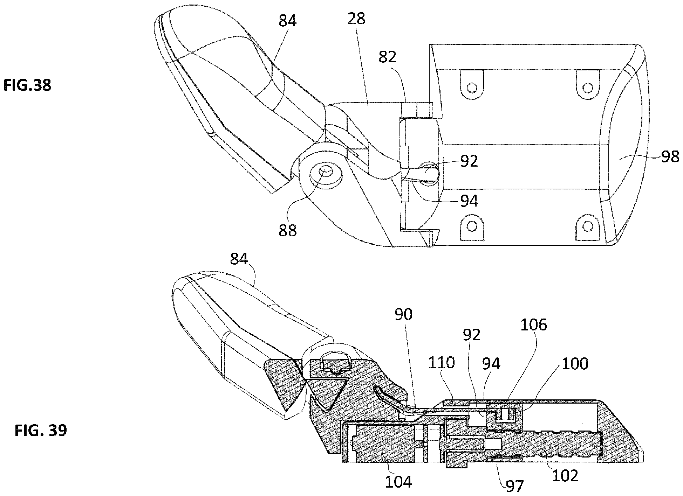

A significant difference between the thumb and finger digits is the need for the thumb to rotate along two non-parallel axes. That is to say, instead of rotating about parallel pivotal axes (as defined for the finger digit phalange sections by pivot pins 20 and 22), the thumb proximal phalange section 78 pivots on an axis defined by joint 82, perpendicular to the linear actuator screw axis, but the distal thumb phalange section 84 pivots about an axis which is not parallel to that of j oint 82. The reason for this is that the thumb rotates through multiple axes to curl around objects and grip them. Accordingly, an internal channel 110 (FIG. 39) guides the strap member from the linear actuator through the proximal thumb phalange member 78 to the distal thumb phalange member 84 which is not in line with the linear actuator. The properties of the strap member tendon, including useful though limited lateral flexibility as described above, allow for push-pull motion of the thumb digit through multiple pivots that are not aligned. This avoids any need for multiple motors to drive the thumb, as the single linear actuator and thumb-tendon strap member can first rotate the thumb into position about joint 82 and then close thumb about pin 88, resulting in a relatively accurate and capable looking thumb assembly with minimal complexity.

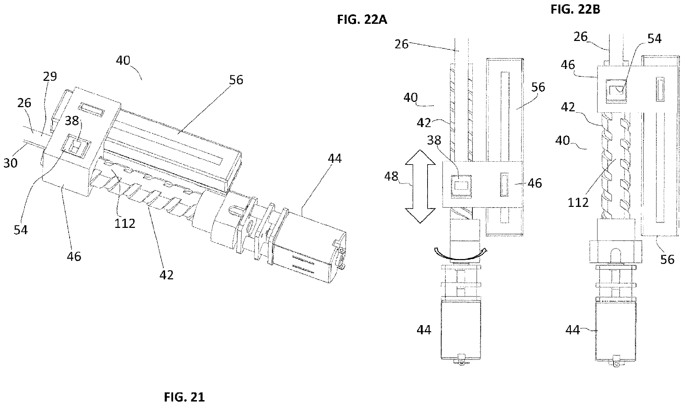

As stated, it is currently preferred to use 3D printing to manufacture most of the components of the described prosthetic hand, apart from the motors, potentiometers and some other ancillary fittings and connections. 3D printing allows advantageous customization while achieving production at relatively low cost. The design of components and especially the orientation of parts has been selected to reduce the amount of support structures required in 3D printing and reduce printing time as well as increase strength and definition. For instance, as shown in the drawings, the lead screws in the linear actuators are configured with opposed longitudinal flat sides 112 (FIG. 22) to allow them to print flat on a build plate along the strongest direction and make them easy to print with good definition along the layer lines. The inner palm support that houses all the components for the linear actuators has also been optimized to reduce support structures and give significant strength to hinges and other features. Finger digit sections have also been designed to print flat on their side which allows for stronger hinges (continuous line of filament). The decorative plates have been designed to be printed on the flat visible face to make it as aesthetic and clean as possible. The inner housing (palm body casing) has been designed to be printed with the wrist opening flat on the build surface to allow more organic shapes with less visible layering. Overall, objectives of the design are to reduce the cost of the devices, reduce print time, provide desired strength of printed parts and lower the number of parts and actuators.

The use of the hand will be readily apparent to persons skilled in the art, being effected by operation of the linear actuator motors, for example (in prosthetic use) under myoelectric control (through leads, not shown, extending externally of the hand) utilizing information supplied from the linear potentiometers and/or other sensors. The motors themselves may be powered by rechargeable batteries. Controlling electronics, microcontrollers and such that interpret the control signals and drive the motors based on positive sensors may also be provided. As stated, the hand structure of the invention may also be used in robotics, not just in prosthetics.

It is to be understood that the invention is not limited to the features and embodiments hereinabove specifically set forth, but may be carried out in other ways without departure from its scope.

## Claims

1. An artificial hand (10), comprising:
(a) a palm body (18);
(b) at least two finger digits (11, 12, 14, 15) extending distally from the palm body (18) and each having a knuckle element (11c, 12c, 14c, 15c) pivotally connected to the palm body (18), and **characterised by** further comprising:
(c) an adductor-abductor plate (60) mounted in the palm body (18) for sliding movement in distal and proximal directions and having diverging slots (68), and an adductor-abductor linear actuator (70) in the palm body (18) for effecting sliding movement of the plate as aforesaid, and wherein each of the knuckles (11c, 12c, 14c, 15c) has an arm (64) extending proximally from the knuckle over the palm body (18) and bearing a peg (66) inserted in one of the slots (68), such that sliding movement of the plate (60) causes the slots (68) to guide the pegs (66) divergently or convergently thereby to rotate the knuckles for adduction or abduction of the finger digits(11, 12, 14, 15).

2. An artificial hand (10) as defined in claim 1, wherein the number of diverging slots (68) is equal to the number of finger digits (11, 12, 14, 15).

3. An artificial hand (10) as defined in any preceding claim, wherein the at least two finger digits (11, 12, 14, 15) further comprise a plurality of successive phalange sections (11a, 11b, 12a, 12b, 14a, 14b, 15a, 15b) pivotally interconnected endwise, the phalange sections (11a, 11b, 12a, 12b, 14a, 14b, 15a, 15b) of each of said finger digits including at least
(i) a proximal phalange section (11a, 12a, 14a, 15a) with a proximal end pivotally connected to the palm body (18) through the knuckle element, and
(ii) a distal phalange section (11b, 12b, 14b, 15b),
wherein axes of pivotal interconnection of successive phalange sections (11a, 11b, 12a, 12b, 14a, 14b, 15a, 15b) and of pivotal connection of the proximal phalange section (11a, 12a, 14a, 15a) to the knuckle element (11c, 12c, 14c, 15c) are substantially parallel and oriented such that the phalange sections (11a, 11b, 12a, 12b, 14a, 14b, 15a, 15b) are pivotally movable relative to each other and to the knuckle element (11c, 12c, 14c, 15c) in simulation of movement of a human finger between extended and inwardly curled positions relative to a human palm; and
wherein the knuckle element (11c, 12c, 14c, 15c) is connected to the palm body (18) for pivotal movement relative thereto in a plane parallel to the axis of pivotal movement of the proximal phalange section (11a, 12a, 14a, 15a) relative to the knuckle element.

4. An artificial hand (10) as defined in claim 3, comprising:
a tendon for each finger digit, comprising an elongated flexible strap (26) member extending lengthwise through the successive phalange sections (11a, 11b, 12a, 12b, 14a, 14b, 15a, 15b) and offset inwardly from said axes with respect to a direction of finger curling, the strap member having a distal end (32) anchored to the distal phalange section, the strap member having flexibility such that a force applied to move a proximal end (38) of the strap member longitudinally relative to the phalange sections (11a, 11b, 12a, 12b, 14a, 14b, 15a, 15b) of the finger digit in each of two opposed directions effects movement of the phalange sections (11a, 11b, 12a, 12b, 14a, 14b, 15a, 15b) pivotally relative to each other and to the palm body (18) between positions respectively simulating said extended and inwardly curled positions; and
(d) a finger-digit linear actuator (40) within the palm body (18) and connected to the proximal end (38) of each strap member for selectively applying said force thereto in each of said two opposed directions.

5. An artificial hand (10) as defined in claim 4, wherein the palm body (18) further includes a rigid casing (74) for receiving and enclosing said finger-digit linear actuators (40), said adductor-abductor linear actuator (70), said adductor-abductor plate (60) and said arms.

6. An artificial hand (10) as defined in claim 4 or 5, having four of said finger digits respectively corresponding to the index, middle, ring and little fingers of a human hand, and first, second and third finger-digit linear actuators (40) disposed side-by-side in the palm body (18) with the first and second respectively connected to the proximal ends (38) of the tendons of the index and middle finger digits and the third connected to the proximal ends (38) of the tendons of the ring and little finger digits.

7. An artificial hand (10) as defined in any one of claims 4 to 6, wherein the elongated flexible strap (26) member has greater flexibility in a longitudinal direction than in a transverse direction.

8. An artificial hand (10) as defined in any of claims 4 to 7, wherein said finger-digit linear actuator (40) includes a bidirectionally movable driver member (106) to which the proximal end (38) of the strap member is fixed, the driver member (106) having a path of travel entirely within the palm body (18) between limits corresponding to positions of the phalange sections (11a, 11b, 12a, 12b, 14a, 14b, 15a, 15b) respectively simulating said extended and inwardly curled positions.

9. An artificial hand (10) as defined in any of claims 7 and 8, wherein the finger-digit linear actuator (40) comprises a lead screw (102), a motor (104) for rotating the lead screw (102) in each of two opposed directions, and a driver member (106) threaded on the lead screw (102) for longitudinal movement therealong in each of two opposed directions dependent on the direction of rotation of the lead screw, the driver member (106) being anchored to a proximal end (38) of the strap member for moving the strap member longitudinally through the phalange sections (11a, 11b, 12a, 12b, 14a, 14b, 15a, 15b) as aforesaid.

10. An artificial hand (10) as defined in claim 9, including a detector for determining the longitudinal position of the driver member (106) along the lead screw.

11. An artificial hand (10) as defined in any preceding claim, further comprising a thumb digit (16) extending laterally from the palm body (18) and having
(i) a proximal thumb phalange section (78) with a proximal end pivotally connected to the palm body (18) for rotation relative thereto about a first thumb axis and
(ii) a distal thumb phalange section (84) with a proximal end pivotally connected to the distal end of the proximal thumb phalange section (78) for rotation relative thereto about a second thumb axis non-parallel to the first thumb axis,
such that the thumb phalange sections (78, 84) are pivotally movable relative to each other and to the palm body (18) in simulation of movement of a human thumb relative to a human palm;
(c) a tendon for said thumb digit, comprising an elongated flexible flat thumb strap member (90) extending lengthwise through the successive thumb phalange sections with major flat surfaces offset inwardly from said first and second thumb axes with respect to a direction of thumb bending, the strap member having a distal end (32) anchored to the distal thumb phalange section; said successive thumb phalange sections cooperatively defining a non-rectilinear path for guiding the thumb strap member longitudinally therethrough, and
(d) a thumb linear actuator (97) mounted on the palm body (18) and connected to a proximal end (100) of the thumb strap member (90) for moving the thumb strap member (90) longitudinally through the thumb phalange sections (78, 84) in each of two opposed directions thereby to move the thumb phalange sections (78, 84) pivotally relative to each other and to the palm body (18) between positions respectively simulating said extended and inwardly curled positions of a human thumb relative to a human palm.

12. An artificial hand (10) as defined in claim 11, wherein the thumb linear actuator (97) comprises a lead screw (102) extending transversely across the palm body, a motor (104) for rotating the lead screw (102) in each of two opposed directions, and a driver member (106) threaded on the lead screw (102) for longitudinal movement therealong in each of two opposed directions dependent on the direction of rotation of the lead screw, the driver member (106) being anchored to a proximal end (100) of the thumb strap member for moving the thumb strap member (90) longitudinally through the thumb phalange sections as aforesaid.

13. An artificial hand (10) as defined in claim 11 or 12, wherein the palm body (18) further includes a rigid casing (74) for receiving and enclosing said finger-digit linear actuator means (40), said adductor-abductor linear actuator (70), said adductor-abductor plate (60) and said arms, and wherein the thumb digit (16) includes a housing containing the thumb linear actuator (97) and mounted on a palm face of the casing.

14. An artificial hand (10) as defined in any one of claims 11 to 13, wherein one of said thumb phalange sections has a channel (110) for receiving and guiding the thumb strap member (90) in said non-rectilinear path.

## Patentansprüche

1. Künstliche Hand (10), umfassend:
(a) einen Handflächenkörper (18);
(b) wenigstens zwei Fingerglieder (11, 12, 14, 15), die sich distal von dem Handflächenkörper (18) erstrecken und jeweils ein Knöchelelement (11c, 12c, 14c, 15c) aufweisen, das schwenkbar mit dem Handflächenkörper (18) verbunden ist, und **gekennzeichnet durch** weiteres Umfassen von:
(c) einer Adduktor-Abduktor-Platte (60), die in dem Handflächenkörper (18) für eine Gleitbewegung in distaler und proximaler Richtung montiert ist und divergierende Schlitze (68) aufweist, und einem linearen Adduktor-Abduktor-Aktuator (70) in dem Handflächenkörper (18) zum Bewirken einer Gleitbewegung der Platte wie vorstehend erwähnt, und wobei jeder der Knöchel (11c, 12c, 14c, 15c) einen Arm (64) aufweist, der sich proximal von dem Knöchel über den Handflächenkörper (18) erstreckt und einen Zapfen (66) trägt, der in einen der Schlitze (68) eingesetzt ist, sodass eine Gleitbewegung der Platte (60) bewirkt, dass die Schlitze (68) die Zapfen (66) divergent oder konvergent führen, wodurch die Knöchel für die Adduktion oder Abduktion der Fingerglieder (11, 12, 14, 15) gedreht werden.

2. Künstliche Hand (10) nach Anspruch 1, wobei die Anzahl der divergierenden Schlitze (68) gleich der Anzahl der Fingerglieder (11, 12, 14, 15) ist.

3. Künstliche Hand (10) nach einem der vorhergehenden Ansprüche, wobei die wenigstens zwei Fingerglieder (11, 12, 14, 15) ferner eine Mehrzahl von aufeinanderfolgenden Phalangenbereichen (11a, 11b, 12a, 12b, 14a, 14b, 15a, 15b) umfassen, die endseitig schwenkbar miteinander verbunden sind, wobei die Phalangenbereiche (11a, 11b, 12a, 12b, 14a, 14b, 15a, 15b) jedes der Fingerglieder wenigstens beinhalten
(i) einen proximalen Phalangenbereich (11a, 12a, 14a, 15a) mit einem proximalen Ende, das schwenkbar mit dem Handflächenkörper (18) durch das Knöchelelement verbunden ist, und
(ii) einen distalen Phalangenbereich (11b, 12b, 14b, 15b),
wobei die Achsen der schwenkbaren Zwischenverbindung der aufeinanderfolgenden Phalangenbereiche (11a, 11b, 12a, 12b, 14a, 14b, 15a, 15b) und der schwenkbaren Verbindung des proximalen Phalangenabschnitts (11a, 12a, 14a, 15a) mit dem Knöchelelement (11c, 12c, 14c, 15c) im Wesentlichen parallel und derart ausgerichtet sind, dass die Phalangenbereiche (11a, 11b, 12a, 12b, 14a, 14b, 15a, 15b) relativ zueinander und zu dem Knöchelelement (11c, 12c, 14c, 15c) schwenkbar beweglich sind, um eine Bewegung eines menschlichen Fingers zwischen ausgestreckten und nach innen gekrümmten Positionen relativ zu einer menschlichen Handfläche zu simulieren; und
wobei das Knöchelelement (11c, 12c, 14c, 15c) mit dem Handflächenkörper (18) für eine schwenkbare Bewegung relativ dazu auf einer Ebene parallel zu der Achse der schwenkbaren Bewegung des proximalen Phalangenbereichs (11a, 12a, 14a, 15a) relativ zu dem Knöchelelement verbunden ist.

4. Künstliche Hand (10) nach Anspruch 3, umfassend:
eine Sehne für jedes Fingerglied, umfassend ein langgestrecktes, flexibles Bandelement (26), das sich in Längsrichtung durch die aufeinanderfolgenden Phalangenbereiche (11a, 11b, 12a, 12b, 14a, 14b, 15a, 15b) erstreckt und von den Achsen nach innen in Bezug auf eine Richtung des Fingerkrümmens versetzt ist, wobei das Bandelement ein distales Ende (32) aufweist, das mit dem distalen Phalangenbereich verankert ist, wobei das Bandelement eine Flexibilität aufweist, sodass eine Kraft, die zum Bewegen eines proximalen Endes (38) des Bandelements in Längsrichtung relativ zu den Phalangenbereichen (11a, 11b, 12a, 12b, 14a, 14b, 15a, 15b) des Fingerglieds in j eweils zwei entgegengesetzte Richtungen angewendet wird, eine Bewegung der Phalangenbereiche (11a, 11b, 12a, 12b, 14a, 14b, 15a, 15b) schwenkbar relativ zueinander und zu dem Handflächenkörper (18) zwischen Positionen bewirkt, die die ausgestreckten beziehungsweise nach innen gekrümmten Positionen simulieren; und
(d) einen linearen Fingergliedaktuator (40) innerhalb des Handflächenkörpers (18) und mit dem proximalen Ende (38) jedes Bandelements verbunden, um selektiv die Kraft darauf in jeder der zwei entgegengesetzten Richtungen aufzuwenden.

5. Künstliche Hand (10) nach Anspruch 4, wobei der Handflächenkörper (18) ferner ein starres Gehäuse (74) zum Aufnehmen und zum Umschließen der linearen Fingergliederaktuatoren (40), des linearen Adduktor-Absorber-Aktuators (70), der Adduktor-Absorber-Platte (60) und der Arme beinhaltet.

6. Künstliche Hand (10) nach Anspruch 4 oder 5, mit vier der Fingerglieder, die dem Zeige-, Mittel-, Ring- beziehungsweise kleinen Finger einer menschlichen Hand entsprechen, und einem ersten, zweiten und dritten linearen Fingergliedaktuator (40), die Seite an Seite in dem Handflächenkörper (18) angeordnet sind, wobei der erste und der zweite jeweilig mit den proximalen Enden (38) der Sehnen der Zeige- und Mittelfingerglieder verbunden sind und der dritte mit den proximalen Enden (38) der Sehnen der Ring- und kleinen Fingerglieder verbunden ist.

7. Künstliche Hand (10) nach einem der Ansprüche 4 bis 6, wobei das langgestreckte flexible Band- (26) Element in einer Längsrichtung eine größere Flexibilität aufweist als in einer Querrichtung.

8. Künstliche Hand (10) nach einem der Ansprüche 4 bis 7, wobei der lineare Fingergliederaktuator (40) ein bidirektional bewegliches Antriebselement (106) beinhaltet, an dem das proximale Ende (38) des Bandelements befestigt ist, wobei das Antriebselement (106) einen Bewegungsweg aufweist, der vollständig innerhalb des Handflächenkörpers (18) zwischen Begrenzungen liegt, die den Positionen der Phalangenbereiche (11a, 11b, 12a, 12b, 14a, 14b, 15a, 15b) entsprechen, die die ausgestreckten beziehungsweise nach innen gekrümmten Positionen simulieren.

9. Künstliche Hand (10) nach einem der Ansprüche 7 und 8, wobei der lineare Fingergliedaktuator (40) eine Leitspindel (102), einen Motor (104) zum Drehen der Leitspindel (102) in jede von zwei entgegengesetzten Richtungen und ein Antriebselement (106) umfasst, das auf die Leitspindel (102) für eine Längsbewegen daran entlang in jeder von zwei entgegengesetzten Richtungen in Abhängigkeit von der Drehrichtung der Leitspindel gewindet ist, wobei das Antriebselement (106) an einem proximalen Ende (38) des Bandelements verankert ist, um das Bandelement in Längsrichtung durch die Phalangenbereiche (11a, 11b, 12a, 12b, 14a, 14b, 15a, 15b) zu bewegen, wie vorstehend beschrieben.

10. Künstliche Hand (10) nach Anspruch 9, beinhaltend einen Detektor zum Bestimmen der Längsposition des Antriebselements (106) entlang der Leitspindel.

11. Künstliche Hand (10) nach einem der vorhergehenden Ansprüche, die ferner ein Daumenglied (16) umfasst, das sich seitlich von dem Handflächenkörper (18) erstreckt und aufweist:
(i) einen proximalen Daumenphalangenbereich (78) mit einem proximalen Ende, das schwenkbar mit dem Handflächenkörper (18) für die Drehung relativ dazu um eine erste Daumenachse verbunden ist, und
(ii) einen distalen Daumenphalangenbereich (84) mit einem proximalen Ende, das schwenkbar mit dem distalen Ende des proximalen Daumenphalangenbereichs (78) für die Drehung relativ dazu um eine zweite Daumenachse verbunden ist, die nicht parallel zu der ersten Daumenachse verläuft,
sodass die Daumenphalangenbereiche (78, 84) relativ zueinander und zu dem Handflächenkörper (18) schwenkbar sind, um die Bewegung eines menschlichen Daumens relativ zu einer menschlichen Handfläche zu simulieren;
(c) eine Sehne für das Daumenglied, umfassend ein längliches, flexibles, flaches Daumenbandelement (90), das sich in Längsrichtung durch die aufeinanderfolgenden Daumenphalangenbereiche erstreckt, wobei große, flache Oberflächen nach innen von den ersten und zweiten Daumenachsen in Bezug auf eine Richtung der Daumenbeugung versetzt sind, wobei das Bandelement ein distales Ende (32) aufweist, das an dem distalen Daumenphalangenbereich verankert ist; wobei die aufeinanderfolgenden Daumenphalangenbereiche zusammenwirkend einen nicht geradlinigen Weg für die Führung des Daumenbandelementes in Längsrichtung dahindurch definieren, und
(d) einen linearen Daumenaktuator (97), der an dem Handflächenkörper (18) montiert und mit einem proximalen Ende (100) des Daumenbandelements (90) zum Bewegen des Daumenbandelements (90) in Längsrichtung durch die Daumenphalangen (78, 84) in jeder der zwei entgegengesetzten Richtungen verbunden ist, wodurch die Daumenphalangenbereiche (78, 84) relativ zueinander und zu dem Handflächenkörper (18) zwischen Positionen schwenkbar bewegt werden, die die ausgestreckten beziehungsweise nach innen gekrümmten Positionen eines menschlichen Daumens relativ zu einer menschlichen Handfläche simulieren.

12. Künstliche Hand (10) nach Anspruch 11, wobei der lineare Daumenaktuator (97) eine Leitspindel (102), die sich quer über den Handflächenkörper erstreckt, einen Motor (104) zum Drehen der Leitspindel (102) in jede von zwei entgegengesetzten Richtungen und ein Antriebselement (106), das auf die Leitspindel (102) gewindet ist, für eine Bewegung in Längsrichtung daran entlang in jeder der zwei entgegengesetzten Richtungen abhängig von der Drehrichtung der Leitspindel, wobei das Antriebselement (106) an einem proximalen Ende (100) des Daumenbandelements zum Bewegen des Daumenbandelements (90) in Längsrichtung durch die Daumenphalangenbereiche verankert ist, wie vorstehend beschrieben.

13. Künstliche Hand (10) nach Anspruch 11 oder 12, wobei der Handflächenkörper (18) ferner ein starres Gehäuse (74) zum Aufnehmen und zum Umschließen des linearen Fingerglied-Aktuatormittels (40), des Adduktor-Abduktor-Aktuators (70), der Adduktor-Absorber-Platte (60) und der Arme beinhaltet, wobei das Daumenglied (16) ein Gehäuse beinhaltet, das den linearen Daumengliedaktuator (97) beinhaltet und an einer Handflächenfläche des Gehäuses montiert ist.

14. Künstliche Hand (10) nach einem der Ansprüche 11 bis 13, wobei einer der Daumenphalangenbereiche einen Kanal (110) zum Aufnehmen und Führen des Daumenbandelements (90) in einem nicht geradlinigen Weg aufweist.

## Revendications

1. Main artificielle (10), comprenant :
(a) un corps de paume (18) ;
(b) au moins deux membres doigts (11, 12, 14, 15) s'étendant distalement depuis le corps de paume (18) et chacun ayant un élément d'articulation (11c, 12c, 14c, 15c) relié de façon pivotante au corps de paume (18), et **caractérisé en ce qu'**elle comprend en outre :
(c) une plaque d'adducteur-abducteur (60) montée dans le corps de paume (18) pour mouvement coulissant dans des directions distale et proximale et ayant des fentes divergentes (68), et un actionneur linéaire d'adducteur-abducteur (70) dans le corps de paume (18) pour effectuer un mouvement coulissant de la plaque telle que susdite, et dans laquelle chacune des articulations (11c, 12c, 14c, 15c) a un bras (64) s'étendant proximalement depuis l'articulation par-dessus le corps de paume (18) et supportant un tenon (66) inséré dans une des fentes (68), de manière telle que le mouvement coulissant de la plaque (60) amène les fentes (68) à guider les tenons (66) de façon divergente ou convergente, pour ainsi entraîner en rotation les articulations pour adduction ou abduction des membres doigts (11, 12, 14, 15).

2. Main artificielle (10) telle que définie dans la revendication 1, dans laquelle le nombre de fentes divergentes (68) est égal au nombre de membres doigts (11, 12, 14, 15).

3. Main artificielle (10) telle que définie dans une quelconque revendication précédente, dans laquelle les au moins deux membres doigts (11, 12, 14, 15) comprennent en outre une pluralité de sections phalanges successives (11a, 11b, 12a, 12b, 14a, 14b, 15a, 15b) reliées mutuellement de façon pivotante à l'extrémité, les sections phalanges (11a, 11b, 12a, 12b, 14a, 14b, 15a, 15b) de chacun desdits membres doigts incluant au moins
(i) une section phalange proximale (11a, 12a, 14a, 15a) avec une extrémité proximale reliée de façon pivotante au corps de paume (18) par l'intermédiaire de l'élément d'articulation, et
(ii) une section phalange distale (11b, 12b, 14b, 15b),
dans laquelle des axes de liaison mutuelle pivotante de sections phalanges successives (11a, 11b, 12a, 12b, 14a, 14b, 15a, 15b) et de liaison pivotante de la section phalange proximale (11a, 12a, 14a, 15a) à l'élément d'articulation (11c, 12c, 14c, 15c) sont sensiblement parallèles et orientés de manière telle que les sections phalanges (11a, 11b, 12a, 12b, 14a, 14b, 15a, 15b) sont mobiles de façon pivotante les unes relativement aux autres et à l'élément d'articulation (11c, 12c, 14c, 15c) en simulation de mouvement d'un doigt humain entre des positions étendue et fléchie vers l'intérieur relativement à une paume humaine ; et
dans laquelle l'élément d'articulation (11c, 12c, 14c, 15c) est relié au corps de paume (18) pour mouvement pivotant relativement à celui-ci dans un plan parallèle à l'axe de mouvement pivotant de la section phalange proximale (11a, 12a, 14a, 15a) relativement à l'élément d'articulation.

4. Main artificielle (10) telle que définie dans la revendication 3, comprenant :
un tendon pour chaque membre doigt, comprenant un organe sangle flexible allongé (26) s'étendant en longueur à travers les sections phalanges successives (11a, 11b, 12a, 12b, 14a, 14b, 15a, 15b) et décalé vers l'intérieur par rapport auxdits axes quant à une direction de flexion de doigt, l'organe sangle ayant une extrémité distale (32) encrée à la section phalange distale, l'organe sangle ayant une flexibilité de manière telle qu'une force appliquée pour mouvoir une extrémité proximale (38) de l'organe sangle longitudinalement relativement aux sections phalanges (11a, 11b, 12a, 12b, 14a, 14b, 15a, 15b) du membre doigt dans chacune de deux directions opposées effectue le mouvement des sections phalanges (11a, 11b, 12a, 12b, 14a, 14b, 15a, 15b) de façon pivotante les unes relativement aux autres et au corps de paume (18) entre des positions simulant respectivement lesdites positions étendue et fléchie vers l'intérieur ; et
(d) un actionneur linéaire de membre doigt (40) à l'intérieur du corps de paume (18) et relié à l'extrémité proximale (38) de chaque organe sangle pour appliquer sélectivement ladite force sur celui-ci dans chacune desdites deux directions opposées.

5. Main artificielle (10) telle que définie dans la revendication 4, dans laquelle le corps de paume (18) inclut en outre un boîtier rigide (74) pour recevoir et enfermer ledit actionneurs linéaires de membres doigts (40), ledit actionneur linéaire d'adducteur-abducteur (70), ladite plaque d'adducteur-abducteur (60) et lesdits bras.

6. Main artificielle (10) telle que définie dans la revendication 4 ou 5, ayant quatre desdits membres doigts correspondant respectivement à l'index, au majeur, à l'annulaire, et à l'auriculaire d'une main humaine, et des premier, deuxième, et troisième actionneurs linéaires de membres doigts ( 40) disposés côte-à-côte dans le corps de paume (18) avec les premier et deuxième respectivement reliés aux extrémités proximales (38) des tendons des membres doigts index et majeur et le troisième relié aux extrémités proximales (38) des tendons des membres doigts annulaire et auriculaire.

7. Main artificielle (10) telle que définie dans l'une quelconque de revendications 4 à 6, dans laquelle l'organe sangle flexible allongé (26) a une plus grande flexibilité dans une direction longitudinale que dans une direction transversale.

8. Main artificielle (10) telle que définie dans de quelconques des revendications 4 à 7, dans laquelle ledit actionneur linéaire de membre doigt ( 40) inclut un organe d'entraînement mobile de façon bidirectionnelle (106) auquel l'extrémité proximale (38) de l'organe sangle est fixée, l'organe d'entraînement (106) ayant une voie de déplacement entièrement à l'intérieur du corps de paume (18) entre des limites correspondant à des positions des sections phalanges (11a, 11b, 12a, 12b, 14a, 14b, 15a, 15b) simulant respectivement lesdites positions étendue et fléchie vers l'intérieur.

9. Main artificielle (10) telle que définie dans de quelconques des revendications 7 et 8, dans laquelle l'actionneur linéaire de membre doigt (40) comprend une vis mère (102), un moteur (104) pour entraîner en rotation la vis mère (102) dans chacune de deux directions opposées, et un organe d'entraînement (106) vissé sur la vis mère (102) pour mouvement longitudinal le long de celle-ci dans chacune de deux directions opposées en fonction de la direction de rotation de la vis mère, l'organe d'entraînement (106) étant encré à une extrémité proximale (38) de l'organe sangle pour mouvoir l'organe sangle longitudinalement à travers les sections phalanges (11a, 11b, 12a, 12b, 14a, 14b, 15a, 15b) telles que susdites.

10. Main artificielle (10) telle que définie dans la revendication 9, incluant un détecteur pour déterminer la position longitudinale de l'organe d'entraînement (106) le long de la vis mère.

11. Main artificielle (10) telle que définie dans une quelconque revendication précédente, comprenant en outre un membre pouce (16) s'étendant latéralement depuis le corps de paume (18) et ayant
(i) une section phalange de pouce proximale (78) avec une extrémité proximale reliée de façon pivotante au corps de paume (18) pour rotation relativement à celle-ci autour d'un premier axe de pouce et
(ii) une section phalange de pouce distale (84) avec une extrémité proximale reliée de façon pivotante à l'extrémité distale de la section phalange de pouce proximale (78) pour rotation relativement à celle-ci autour d'un second axe de pouce non parallèle au premier axe de pouce,
de manière telle que les sections phalanges de pouce (78, 84) sont mobiles de façon pivotante l'une relativement à l'autre et au corps de paume (18) en simulation de mouvement d'un pouce humain relativement à une paume humaine ;
(c) un tendon pour ledit membre pouce, comprenant un organe sangle de pouce plat flexible allongé (90) s'étendant en longueur à travers les sections phalanges de pouce successive avec des surfaces plates principales décalées vers l'intérieur quand auxdits premier et second axes de pouce par rapport à une direction de pliage de pouce, l'organe sangle ayant une extrémité distale (32) encrée à la section phalange de pouce distale ; lesdites sections phalanges de pouce successives définissant en coopération une voie non rectiligne pour guider l'organe sangle de pouce longitudinalement à travers celui-ci, et
(d) un actionneur linéaire de pouce (97) monté sur le corps de paume (18) et relié à une extrémité proximale (100) de l'organe sangle de pouce (90) pour mouvoir l'organe sangle de pouce (90) longitudinalement à travers les sections phalanges de pouce (78, 84) dans chacune de deux directions opposées pour ainsi mouvoir les sections phalanges de pouce (78, 84) de façon pivotante l'une relativement à l'autre et au corps de paume (18) entre des positions simulant respectivement lesdites positions étendue et fléchie vers l'intérieur d'un pouce humain relativement à une paume humaine.

12. Main artificielle (10) telle que définie dans la revendication 11, dans laquelle l'actionneur linéaire de pouce (97) comprend une vis mère (102) s'étendant transversalement à travers le corps de paume, un moteur (104) pour entraîner en rotation la vis mère (102) dans chacune de deux directions opposées, et un organe d'entraînement (106) vissé sur la vis mère (102) pour mouvement longitudinal le long de celle-ci dans chacune de deux directions opposées en fonction de la direction de rotation de la vis mère, l'organe d'entraînement (106) étant encré à une extrémité proximale (100) de l'organe sangle de pouce pour mouvoir l'organe sangle de pouce (90) longitudinalement à travers les sections phalanges de pouce telles que susdites.

13. Main artificielle (10) telle que définie dans la revendication 11 ou 12, dans laquelle le corps de paume (18) en outre inclut un boîtier rigide (74) pour recevoir et enfermer ledit moyen actionneur linéaire de membre doigt (40), ledit actionneur linéaire d'adducteur-abducteur (70), ladite plaque d'adducteur-abducteur (60) et lesdits bras, et dans laquelle le membre pouce (16) inclut un logement contenant l'actionneur linéaire de pouce (97) et monté sur une face de paume du boîtier.

14. Main artificielle (10) telle que définie dans l'une quelconque de revendications 11 à 13, dans laquelle une desdites sections phalanges de pouce a une gorge (110) pour recevoir et guider l'organe sangle de pouce (90) dans ladite voie non rectiligne.
